# EUROPEAN PATENT SPECIFICATION

(11) **EP 4 114 305 B1**
(45) Date of publication and mention of the grant of the patent: **17.04.2024**
(21) Application number: 21706153.0
(22) Date of filing: 26.02.2021
(51) Int. Cl.: A61F 2/16

(54) **PACKAGE FOR RETAINING AND DISPENSING A DELIVERY SHAFT ASSEMBLY FOR DELIVERY OF AN IMPLANT**
VERPACKUNG ZUM HALTEN UND ABGEBEN EINER ABGABESCHAFTANORDNUNG ZUR ABGABE EINES IMPLANTATS
EMBALLAGE POUR RETENIR ET DISTRIBUER UN ENSEMBLE TIGE D'ADMINISTRATION POUR L'ADMINISTRATION D'UN IMPLANT

(30) Priority: 27.02.2020 EP 20159797
(43) Date of publication of application: 11.01.2023
(73) Proprietor: iSTAR Medical SA, 1300 Wavre (BE)
(72) Inventor: DETRY, Benoit, 4432 Alleur (BE); BOUHY, Isabelle, 5060 Tamines (BE); UHRLANDT, Stefan, 22587 Hamburg (DE); GIROD, Adrien Bernard, 2502 Biel (CH)
(74) Representative: De Clercq & Partners
(86) International application number: PCT/EP2021/054858
(87) International publication number: WO 2021/170812

(56) References cited:
- WO-A1-2005/110289
- WO-A1-2016/011191
- WO-A1-2017/108498
- WO-A1-2017/182508
- NL-C2- 2 010 675

## Description

### Field of the invention

Provided herein is a package for retaining and dispensing a delivery shaft assembly for delivery of an implant for implantation in a subject.

### Background to the invention

Implants are often used to treat conditions where medicinal treatment has become ineffective or is not available. Techniques for placement of an implant typically rely on an inserter tool that temporarily holds the implants and provides an elongated reach for placement at the implantation site and a mechanism for ejection from the inserter. Implants can be very small, for instance, implants for the treatment of glaucoma typically have a length of 5 mm and a width of 1 mm. An example of an implant and inserter too for treatment of glaucoma is disclosed, for instance, in WO 2017/108498.

A package for an ocular implant delivery device is known from the document WO 2016/011191.

A problem in the art is how to store the implant prior to use so that it is in an optimum condition. A small implant can be difficult to load into an inserter just prior to use. Some implants need to be maintained under liquid storage without damage or without being exposed to air pockets.

### Summary

Described herein is a package (100) for holding and dispensing a delivery shaft assembly (200) for delivery of an implant (230), wherein
- the delivery shaft assembly (200) comprises a delivery shaft (220) having a lumen (222) holding the implant (230), and an adapter (210) configured for coupling to an inserter tool (500),
- the inserter tool (500) is provided with a discharge shaft (510) configured to be received by the delivery shaft assembly lumen (222), and with an adapter coupling (520) configured to couple with the adapter (210), wherein the delivery shaft (220) and/or of the discharge shaft (510) is moveable such that the implant (230) is released from the lumen,

the package (100) comprising:
   - a vial (400) and an insert (300) mounted within a vial holding space (410), wherein the insert (300) comprises:
      - a force receiving body (308) configured to support the adapter (210) during coupling to the inserter tool (500),
      - a delivery shaft retaining mechanism (310) configured to releasably retain the delivery shaft,
wherein an application of force by the inserter tool (500) during coupling to the adapter (210) actuates the delivery shaft retaining mechanism (310) to release the retained delivery shaft (220) for withdrawal of the delivery shaft assembly (200) from the insert (300) and vial (400).

The delivery shaft retaining mechanism (310) may comprise a 1st body (312) and 2nd body (314) configured to co-operate to clamp at least a part of the delivery shaft (220).

The 1st body (312) may be disposed in fixed relation to the force-receiving body (308), and the 2nd body (314) is configured to at least partly move upon actuation of the delivery shaft retaining mechanism (310) away from the 1st body (312) thereby releasing the retained delivery shaft (220).

The delivery shaft retaining mechanism (310) may further comprise an actuating pin (316) and a movement guide (318) wherein an application of force by the inserter tool during coupling advances the pin (316) and movement guide (318) from a retain position to a release position, wherein the movement guide (318), slidably coupled to the 2nd body (314), effects movement of the 2nd body (314) from a retain position to a release position.

The movement guide (318) may comprise at least 1 guiding slot (320) having at least a partly cammed path, the 2nd body (314) may comprise at least one protrusion (322) engaged in the guiding slot (320), movement of the movement guide (318) from the retain to release position may effect sliding of the guiding slot (320) and movement of the at least one protrusion (322) along the cammed path thereby separating at least a part of the 2nd body (314) from the 1st body (312).

The 1st body (312) and the 2nd body (314) may be connected by a hinge, preferably by a living hinge. The vial holding space (410) may be configured for retention of a liquid. The vial (400) may have an opening (412), closable by a cap or removable seal.

The 1st body (312) may be provided with a keeper (324), configured to limit movement of the implant (230) in the delivery shaft (220) lumen (222) in the distal end (40) direction. The keeper may comprise a projection configured to at least partly engage with the delivery shaft (220) lumen (222).

The 1st body (312) and 2nd body (314) co-operate to clamp at least a part of the delivery shaft in a clamping region such that the delivery shaft lumen (222) in the clamping region (312a, 314a) is at least partly narrowed, thereby limiting movement of the implant (230) in a proximal (20) direction.

The insert (300) may further comprise one or more fluid displacement bodies (340)

configured to displace (aqueous) solution from vial holding space (410) during coupling of the insert (300) into the vial holding space (410).

The insert (300) may further comprises an overflow outlet (342) configured for passage of the displaced solution from the vial. The package (100) may further comprise the delivery shaft assembly (200) as defined herein.

### Figure Legends

**FIG. 1** view of a delivery shaft assembly as described herein
**FIG. 2** view of a package as described herein.
**FIG. 3** panels A to C are a sequence of views showing actuation of the retaining shaft mechanism and release of the delivery shaft assembly.
**FIG. 4** shows a package as described herein, emphasising clamping parts of the 1^{st} body and 2^{nd} body that narrow the delivery shaft, and showing the keeper.
**FIG. 5** shows a part of an inserter tool as described herein.
**FIG. 6** panels A to C show a sequence of views in which the inserter tool couples with a delivery shaft assembly, and the retaining shaft mechanism is actuated to release of the delivery shaft assembly.
**FIG. 7** panels A and B show the inserter tool in a primed (Panel A) and deployed (Panel B) configuration.
**FIG. 8** depicts a package as described herein further provided with a plurality of liquid displacement bodies.

### Detailed description of invention

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. It is to be understood that other embodiments may be utilised and structural or logical changes may be made without departing from the scope of the present invention. The following detailed description, therefore, is not to be taken in a limiting sense, and the scope of the present invention is defined by the appended claims.

The terms "distal" or "distal to" and "proximal" or "proximal to" are used throughout the specification, and are terms generally understood in the field to mean towards (proximal) or away (distal) from the user's (practitioner's) side of an apparatus. Thus, "proximal" or "proximal to" means towards the user's side and, therefore, away from the subject's (patient's) side. Conversely, "distal" or "distal to" means towards the subject's side and, therefore, away from the user's side. The term "subject" refers to the human or animal receiving the implant. The term "user" refers to the person performing the implantation (e.g. surgeon, specialist, practitioner).

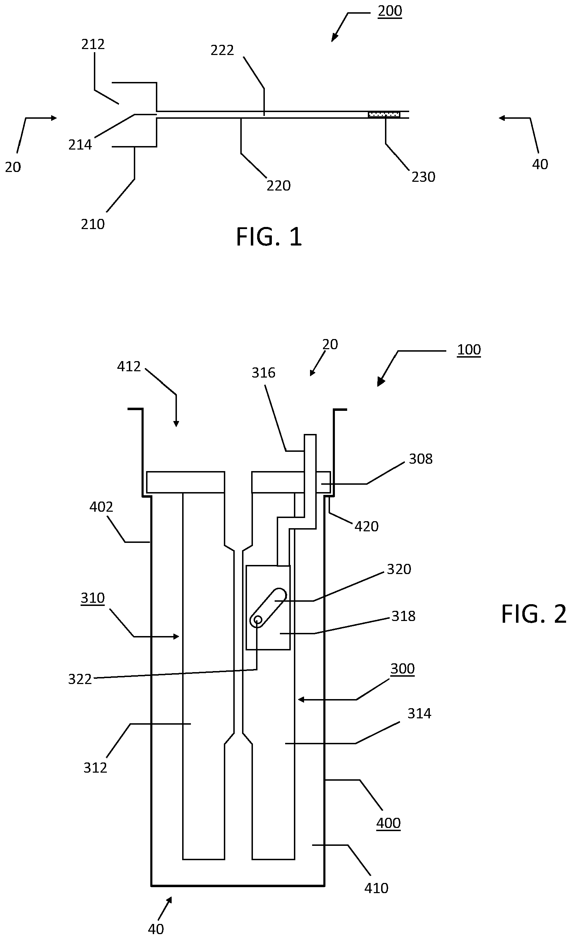

Provided herein is a package (100) for holding and dispensing a delivery shaft assembly (200) for delivery of an implant (230) as exemplified for instance in **FIGs. 1** and **2****.** The package (100) comprises a vial (400) and an insert (300) mounted within a vial holding space (410). The vial holding space (410) is typically provided with a liquid to maintain the implant (230) in a state e.g. hydrated for implantation. The insert (300) comprises a force receiving body (308) configured to support an adapter (210) of delivery shaft assembly (200) during coupling to an inserter tool (500). The insert (300) further comprises a shaft retaining mechanism (310) configured to releasably retain a delivery shaft (220) of the delivery shaft assembly (200). The shaft retaining mechanism (310) is configured such that an application of force by the inserter tool (500) during coupling to the adapter (210) actuates the shaft retaining mechanism (310) to release the retained delivery shaft (220) for withdrawal of the delivery shaft assembly (200) from the insert (300) and vial (400).

Advantageously, the package (100) stabilises the position of the delivery shaft assembly (200) within the vial (400) during transport and storage, preventing unintentional loss of implant from the delivery shaft by transport and handling agitation. By linking a release or retain state of shaft retaining mechanism (310) to an application of force by the inserter tool (500), the delivery shaft assembly (200) is retained in the vial holding space (410) and liquid up until the moment of use, and is released from the insert (300) at the same time as inserter tool couples to it. The package (100) may or may not include the delivery shaft assembly (200). The package (100) may or may not include the inserter tool (500).

The delivery shaft assembly (200) comprises an adapter (210) and a delivery shaft (220). The delivery shaft (220) is provided with a lumen (222) holding the implant (230), and an adapter (210) configured for coupling to the inserter tool (500). The implant (230) is disposed at or towards a distal end (40) of the delivery shaft (220). The adapter (210) is disposed at a proximal end (20) of the delivery shaft (220). The adapter (210) has a body provided with a receiving space (212) for a part of the inserter tool (500) and an aperture (214) connecting the receiving space (212) to a lumen (222) of delivery shaft (220). The lumen (222) is configured for the passage of a discharge shaft (510) of the inserter tool (500) therethrough. The implant (230) may be ejected from the delivery shaft (220) by actuation of the inserter tool (500), thereby delivering the implant (230) to the target. An exemplary delivery shaft assembly (200) is disclosed in WO 2017/108498.

The inserter tool (500) is provided with a discharge shaft (510) configured to be received by the delivery shaft assembly lumen (220). The discharge shaft (510) may be configured abut the implant (230). Movement of the delivery shaft (220) and/or of the discharge shaft (510) causes release of the implant (230) from the lumen (222)

Advancement of the discharge shaft (510) in a distal (40) direction may apply force to the implant (230), causing it to be ejected from the shaft assembly lumen (220). Alternatively or additionally, withdrawal of the delivery shaft (220) relative to a fixed discharge shaft (510) causes the implant (230) to be ejected from the shaft assembly lumen (220).

The inserter tool (500) is provided with an adapter coupling (520) configured to couple with the adapter (210). The coupling between the adapter coupling (520) and adapter (210) may be releasable or non-releasable. A non-releasable coupling may be achieved, for instance, by providing a compliant member on one of the adapter coupling (520) or adapter (210) and a reciprocating stop member on the other, wherein the compliant member slides across the stop member in one direction during couple the parts (520, 210) and the compliant member engages with the stop member in the other sliding direction to prevent release of the parts (520, 210). The mechanism is similar to the one-directional movement of a ratchet mechanism.

Where the implant is ejected by withdrawal of the delivery shaft (220) relative to the fixed discharge shaft (510), the adapter coupling (520) may be slidable relative to the fixed discharge shaft (510). The slidable adapter coupling (520) has a primed and deployed position. In the primed position, the slidable adapter coupling (520) places the adapter (210) and delivery shaft lumen (222) in a distal-most position. The fixed discharge shaft (510) may abut the implant (230) and the delivery shaft lumen (222) may cover the implant (230). In the deployed position, the slidable adapter coupling (520) places the adapter (210) and delivery shaft lumen (222) in a proximal-most position. The discharge shaft (510) abuts the implant (230) and the delivery shaft lumen (222) is withdrawn in a proximal (20) direction and the implant (230) is released. Where the implant is ejected by advancement of the discharge shaft (510), the discharge shaft (510) slidable relative to a fixed adapter coupling (520) applied an ejection force in a distal direction upon the implant (230) causing it to be ejected from the lumen (222).

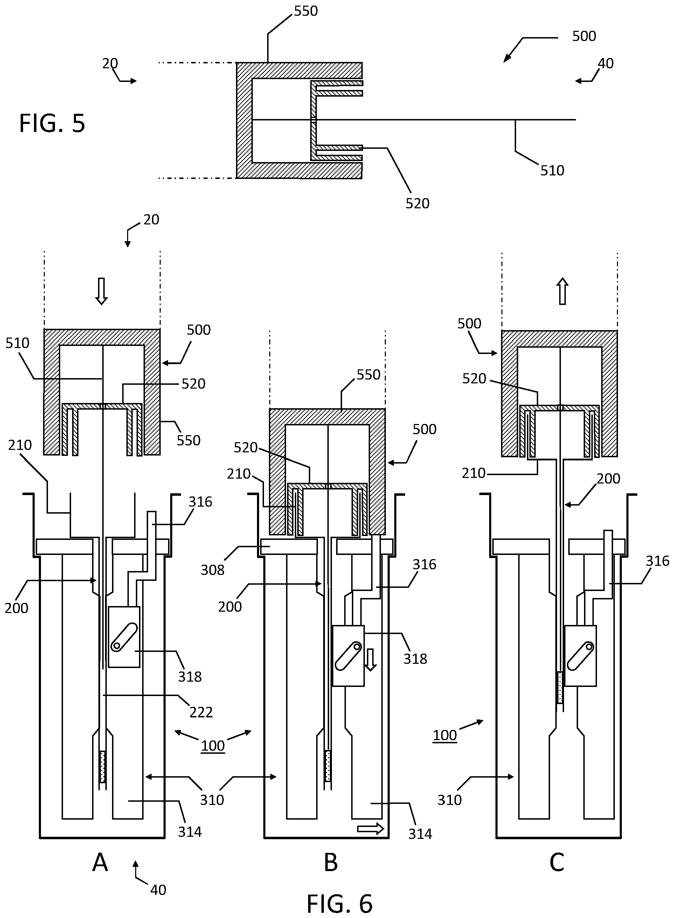

An example of a part of an inserter tool (500) is provided in **FIG. 5****,** showing an inserter housing or chassis (550) (fixed), and a slidable adapter coupling (520) configured to engage with the adapter (210) of the delivery shaft assembly (200). A discharge shaft (510) is disposed in fixed relation to the housing (550). When the adapter (210) is engaged in the adapter coupling (520), the delivery shaft assembly (200) can be lifted from the package (100) while the movement guide (318) is in the release position. In this example, the adapter coupling (520) is slidable relative to the housing (550) and is shown in a primed position; it is appreciated that the other configurations of the inserter tool (500) exist, for instance, in which the adapter coupling (520) is disposed in fixed relation to an inserter tool housing and the discharge shaft (510) is slidable.

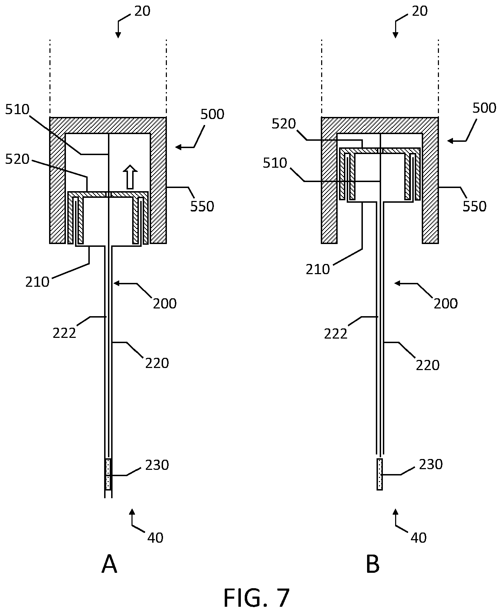

**FIG. 7** illustrates a part of an inserter tool (500) showing an inserter housing or chassis (550) (fixed), and a slidable adapter coupling (520) coupled with the adapter (210) of the delivery shaft assembly (200). In **Panel A,** the inserter tool (500) is shown in the primed position; the adapter coupling (520) is in a distal (40) position. In the primed position, the implant (230) is retained in the delivery shaft lumen (222). In **Panel B,** the inserter tool (500) is shown in the deployed position; the adapter coupling (520) is withdrawn to a proximal (20) position. In the deployed position, the delivery shaft is withdrawn, thereby releasing the implant (230) from the delivery shaft lumen (222).

An exemplary inserter tool (500) is disclosed in WO 2017/108498.

The shaft retaining mechanism (310) may have a retain state (in which the delivery shaft (222) and hence delivery shaft assembly (200) are held fast by the shaft retaining mechanism (310)) and a release state (in which the delivery shaft (222) and hence delivery shaft assembly (200) are free of the insert (300)). Movement from the retain state to the release state is actuated by force of the inserter tool (500) during coupling to the adapter (210). An exemplary shaft retaining mechanism (310) is shown in **FIGs. 2****,** **3A** to **3C, 4.**

The shaft retaining mechanism (310) may comprise a 1st body (312) and 2nd body (314) configured to co-operate (in the retain state) to clamp at least a part of the delivery shaft (222). The delivery shaft (220) may be clamped in fixed relation to the force receiving body.

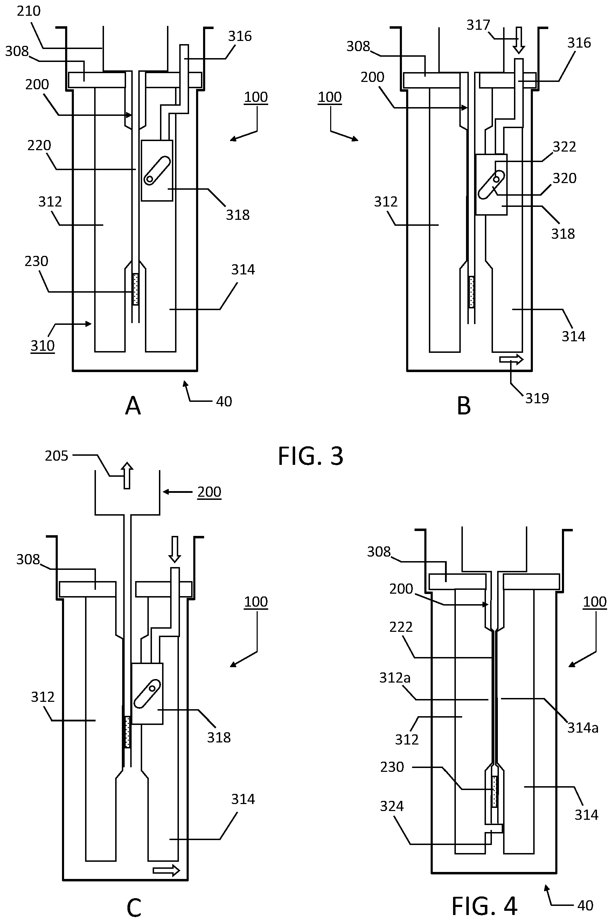

The 1st body (312) and 2nd body (314) may co-operate in the retain state to clamp at least a part of the delivery shaft in a clamping region (312a, 314a) such that the delivery shaft lumen (222) in the clamping region is at least partly narrowed, thereby limiting movement of the implant (230) in a proximal (20) direction. An exemplary clamping region (312a, 314a) narrowing the delivery shaft lumen (222) is shown in **FIG. 4****.**

The 1st body (312) may be disposed in fixed (non-moveable) relation to the force-receiving body (308). The 2nd body (314) may be configured to at least partly move upon actuation of the shaft retaining mechanism (310) away from the 1st body (312) thereby releasing the retained delivery shaft (220).

The 1^{st} body (312) and 2^{nd} body (314) may be longitudinal. The 1^{st} body (312) and 2^{nd} body (314) may be longitudinal in a proximal-distal direction. The 1st body (312) and the 2nd body (314) may be connected by a hinge (*e.g*. living hinge).

The shaft retaining mechanism (310) may further comprising an actuating pin (316) and a movement guide (318). The actuating pin (316) may be attached in fixed relation to the movement guide (318). An application of force by the inserter tool (500) during coupling advances the pin (316) and movement guide (318) from a retain position to a release position. The movement guide (318), slidably coupled to the 2nd body (314), effects movement of the 2nd body (314) from a retain position to a release position.

The movement guide (318) comprises at least 1, or 2 or more (preferably 2) guiding slots (320) having at least a partly cammed path. The cammed path is a linear cam that translates a linear movement in a 1^{st} direction (*e.g*. proximal-distal) to a linear movement in a 2^{nd} direction (*e.g*. perpendicular to the 1^{st} direction).

The 2nd body (314) may comprise a protrusion (322) engaged in a guiding slot (320). There may be one protrusion (322) per guiding slot (320). The protrusion (322) may be fixed relation to the 2nd body. The protrusion (322) may be a follower for the cammed path.

Movement of the movement guide (318) from the retain to release position effects sliding (in a 1^{st} direction) of the guiding slot (320) and movement (in a 2^{nd} direction) of the protrusion (322) along the cammed path thereby separating at least a part of the 2nd body (314) from the 1st body (312).

**FIG. 3** Panels A to C show a package (100) as described herein provided with a delivery shaft assembly (200), illustrating steps of activating the shaft retaining mechanism (310) to move it from a retain state (Panel A) to a release state (Panels B and C). In **Panel A,** the shaft retaining mechanism (310) in the retain state clamps the delivery shaft (220) between the 1st body (312) and 2nd body (314). In **Panel B,** downward force (proximal-distal) (317) applied to the actuating pin (316) displaces the movement guide (318) in a downward direction. The protrusion (322) or follower engaged in the guiding slot (320) is displaced laterally (319), causing movement of the 2^{nd} body in a lateral direction, thereby moving the shaft retaining mechanism (310) to the release state. The delivery shaft assembly (200) is released, and can be withdrawn (205) (**Panel C**) from the insert (300) and hence from the package (100).

**FIG. 6** Panels A to C show a package (100) as described herein provided with a delivery shaft assembly (200), illustrating steps of activating the shaft retaining mechanism (310) to move it from a retain state (Panel A) to a release state (Panels B), coupling of the adapter coupling (520) with the adapter (210) (Panel B), and withdrawal of the delivery shaft assembly (200) from the package (100) (Panel C). In Panel A, the adapter coupling (520) is in a primed position, and the discharge shaft (510) is inserted into the delivery shaft lumen (222). In Panel B, the inserter housing (550) pushes downwards against the actuating pin (316) thereby actuating the movement guide (318) causing movement of the 2^{nd} body (314) to a release position. In Panel B, the inserter adapter coupling (520) in the primed position couples with the deliver shaft assembly adapter (210). The adapter (210) is supported at its distal end by the force receiving surface (308) while the inserter adapter coupling (520) presses downwards (in a distal direction) on the adapter (210) to effect coupling. In Panel C, with inserter adapter coupling (520) still in the primed position and the shaft retaining mechanism (310) in a release position, the delivery shaft assembly (200) can be withdrawn from the package (100) coupled to the inserter tool (500).

The 1st body (312) or second body (314), preferably the 1^{st} body (312) may be provided with a keeper (324). The keeper may be disposed in fixed (non-moveable) relation to the 1^{st} body (312) or 2^{nd} body (314) respectively. The keeper (324) is configured to co-operate with a distal tip of the delivery shaft (220) to limit movement of the implant (230) in the distal end (40) direction. The keeper (324) may comprises a projection (e.g. conical) configured to at least partly engage with the delivery shaft (220) lumen (222). The keeper (324) may be a solid body extending laterally (perpendicular to a proximal-distal direction). **FIG. 4** illustrates a keeper (324) fixed to a distal end (40) of the 1^{st} body (312). The keeper (324) covers a distal end (40) of the delivery shaft lumen (222), and retains the implant (230) by blocking a distal (40) passage of the implant (230). The keeper (324) limits movement of the implant in a distal direction (40).

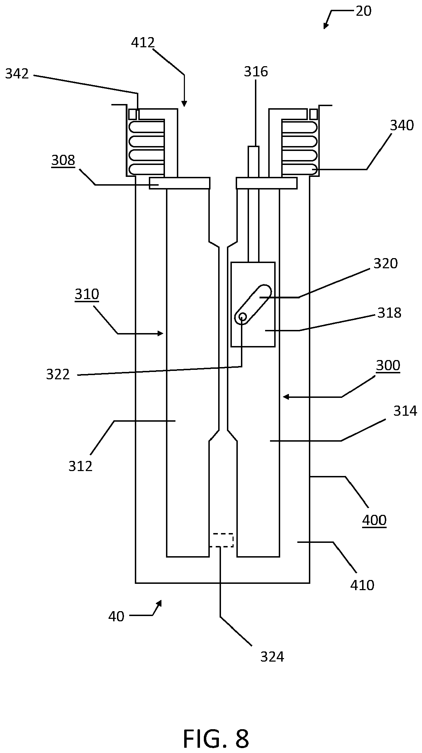

The insert (300) may further comprise one or more liquid displacement bodies (340). A fluid displacement body (340) is configured to displace liquid from vial holding space (410) during placement of the insert (300) during assembly of the package (100). This allows the vial holding space (410) to be filled with a minimum of air bubbles. A liquid displacement body (340) may have a ring-like appearance. The insert (300) may further comprise an overflow outlet (342) configured for passage of the displaced liquid from the vial. The overflow outlet (342) may extend in a proximal direction as a channel into the one or more liquid displacement bodies (340). **FIG. 8** shows a plurality of liquid displacement bodies (340) disposed at a proximal (20) end of the insert (300), and an overflow outlet (342) through which excess liquid is expelled from the holding space (410).

The vial (400) may be formed from a vial body (402) that defines the vial holding space (410). The vial holding space (410) is configured for retention of a liquid *e.g*. aqueous solution, organic solvent. The vial (400) has an opening (412) at the proximal end (20) configured to receive a distal part of the inserter tool (500). The opening (412) may be closed by a cap or removable seal. The vial (400) may be disposed with one or more support members (420) configured to support the insert (300). The support member (420) is configured limit movement of the insert (300) in a distal (40) direction. In particular, when force is applied by the inserter tool (500) to the force receiving surface (308), the support member (420) prevents movement of and damage to the insert (300). The support member (420) may be provided as a ridge on the vial body (402) that engages with the force receiving body (308). An exemplary vial is shown in detail in **FIG. 2****.**

The implant (230) may be any placeable in a subject (animal, human) using an inserter tool. In particular, the implant (230) may be an implant for treatment of glaucoma. In particular, the implant (230) may be an intraocular shunt. The implant (230) may be for implantation between the sclera and the choroid, *i.e.* in the suprachoroidal space. The implant (230) be an implant as described in WO 2017/108498. In particular, the implant may be as described on page 13, lines 1 to page 14, line 5, and/or on page 21, line 25 to page 22 line 21 of WO 2017/108498.

The inserter tool (500) may be an implantation device as described in WO 2017/108498.

The delivery shaft assembly (200) may comprise a one-touch fitting connection element or adaptor (210) as described in WO 2017/108498. For example, WO 2017/108498 describes an implantation device (500) and delivery shaft assembly (200) on pages 26 to page 33.

## Claims

1. A package (100) for holding and dispensing a delivery shaft assembly (200) for delivery of an implant (230) for the treatment of glaucoma wherein
- the delivery shaft assembly (200) comprises a delivery shaft (220) having a lumen (222) holding the implant (230), and an adapter (210) configured for coupling to an inserter tool (500),
- the inserter tool (500) is provided with a discharge shaft (510) configured to be received by the delivery shaft assembly lumen (222), and with an adapter coupling (520) configured to couple with the adapter (210), wherein the delivery shaft (220) and/or of the discharge shaft (510) is moveable such that the implant (230) is released from the lumen,
**characterised in that** the the package (100) comprises:
- a vial (400) and an insert (300) mounted within a vial holding space (410), wherein the insert (300) comprises:
- a force receiving body (308) configured to support the adapter (210) during coupling to the inserter tool (500),
- a delivery shaft retaining mechanism (310) configured to releasably retain the delivery shaft,
wherein an application of force by the inserter tool (500) during coupling to the adapter (210) actuates the delivery shaft retaining mechanism (310) to release the retained delivery shaft (220) for withdrawal of the delivery shaft assembly (200) from the insert (300) and vial (400).

2. The package (100) according to claim 1 wherein the delivery shaft retaining mechanism (310) comprises a 1st body (312) and 2nd body (314) configured to co-operate to clamp at least a part of the delivery shaft (220).

3. The package (100) according to claim 2, wherein the 1st body (312) is disposed in fixed relation to the force-receiving body (308), and the 2nd body (314) is configured to at least partly move upon actuation of the delivery shaft retaining mechanism (310) away from the 1st body (312) thereby releasing the retained delivery shaft (220).

4. The package (100) according to claim 3, wherein the delivery shaft retaining mechanism (310) further comprising an actuating pin (316) and a movement guide (318) wherein an application of force by the inserter tool during coupling advances the pin (316) and movement guide (318) from a retain position to a release position, wherein the movement guide (318), slidably coupled to the 2nd body (314), effects movement of the 2nd body (314) from a retain position to a release position.

5. The package (100) according to claim 4, wherein
- the movement guide (318) comprises at least 1 guiding slot (320) having at least a partly cammed path,
- the 2nd body (314) comprises at least one protrusion (322) engaged in the guiding slot (320),
- movement of the movement guide (318) from the retain to release position effects sliding of the guiding slot (320) and movement of the at least one protrusion (322) along the cammed path thereby separating at least a part of the 2nd body (314) from the 1st body (312).

6. The package (100) according to any of claims 2 to 5, wherein the 1st body (312) and the 2nd body (314) are connected by a hinge, preferably by a living hinge.

7. The package (100) according to any of claims 1 to 6, wherein the vial holding space (410) is configured for retention of a liquid.

8. The package (100) according to any of claims 1 to 7, wherein the vial (400) has an opening (412), closable by a cap or removable seal.

9. The package (100) according to any of claims 1 to 8, wherein 1st body (312) is provided with a keeper (324), configured to limit movement of the implant (230) in the delivery shaft (220) lumen (222) in the distal end (40) direction.

10. The package (100) according to claim 9, wherein the keeper comprises a projection configured to at least partly engage with the delivery shaft (220) lumen (222).

11. The package (100) according to any of claims 2 to 10, wherein the 1st body (312) and 2nd body (314) co-operate to clamp at least a part of the delivery shaft in a clamping region such that the delivery shaft lumen (222) in the clamping region (312a, 314a) is at least partly narrowed, thereby limiting movement of the implant (230) in a proximal (20) direction.

12. The package (100) according to any one of claims 1 to 11, wherein the insert (300) further comprises one or more fluid displacement bodies (340) configured to displace (aqueous) solution from vial holding space (410) during coupling of the insert (300) into the vial holding space (410).

13. The package (100) according to claim 12, whether the insert (300) further comprises an overflow outlet (342) configured for passage of the displaced solution from the vial.

14. The package (100) according to any of claims 1 to 13, further comprising the delivery shaft assembly (200) as defined in claim 1.

## Patentansprüche

1. Packung (100) zum Halten und Abgeben einer Zuführschaftbaugruppe (200) zum Zuführen eines Implantats (230) für die Behandlung von Glaukoma, wobei
- die Zuführschaftbaugruppe (200) einen Zuführschaft (220) mit einem Lumen (222), das das Implantat (230) hält, und einem Adapter (210), der zum Koppeln an ein Einführerwerkzeug (500) ausgestaltet ist, umfasst
- das Einführerwerkzeug (500) mit einem Austrageschaft (510), der zur Aufnahme durch das Zuführschaftbaugruppenlumen (222) ausgestaltet ist, und mit einer Adapterkoppelung (520) versehen ist, die zum Koppeln mit dem Adapter (210) ausgestaltet ist, wobei der Zuführschaft (220) und/oder der Austrageschaft (510) beweglich sind, so dass das Implantat (230) aus dem Lumen freigegeben wird,
**dadurch gekennzeichnet, dass** die Packung (100) Folgendes umfasst:
- ein Vial (400) und einen Einsatz (300), der in einem Vial-Halteraum (410) montiert ist, wobei der Einsatz (300) Folgendes umfasst:
- einen Kraftaufnahmekörper (308), der dazu ausgestaltet ist, den Adapter (210) während des Koppelns an das Einführerwerkzeug (500) zu stützen,
- einen Zuführschafthaltemechanismus (310), der dazu ausgestaltet ist, den Zuführschaft freigebbar zu halten,
wobei durch eine Kraftanwendung durch das Einführerwerkzeug (500) während des Koppelns an den Adapter (210) der Zuführschafthaltemechanismus (310) betätigt wird, um den gehaltenen Zuführschaft (220) freizugeben, um die Zuführschaftbaugruppe (200) aus dem Einsatz (300) und dem Vial (400) herauszuziehen.

2. Packung (100) nach Anspruch 1, wobei der Zuführschafthaltemechanismus (310) einen 1. Körper (312) und einen 2. Körper (314) umfasst, die dazu ausgestaltet sind zusammenzuwirken, um mindestens einen Teil des Zuführschafts (220) zu klemmen.

3. Packung (100) nach Anspruch 2, wobei der 1. Körper (312) in fixierter Beziehung zu dem Kraftaufnahmekörper (308) angeordnet ist und der 2. Körper (314) dazu ausgestaltet ist, sich bei Betätigung des Zuführschafthaltemechanismus (310) mindestens teilweise von dem 1. Körper (312) weg zu bewegen, wodurch der gehaltene Zuführschaft (220) freigegeben wird.

4. Packung (100) nach Anspruch 3, wobei der Zuführschafthaltemechanismus (310) ferner einen Betätigungsstift (316) und eine Bewegungsführung (318) umfasst, wobei der Stift (316) und die Bewegungsführung (318) durch eine Kraftanwendung durch das Einführerwerkzeug während des Koppelns aus einer Halteposition in eine Freigabeposition vorgeschoben werden, wobei die verschiebbar an den 2. Körper (314) gekoppelte Bewegungsführung (318) die Bewegung des 2. Körpers (314) aus einer Halteposition in eine Freigabeposition bewirkt.

5. Packung (100) nach Anspruch 4, wobei
- die Bewegungsführung (318) mindestens 1 Führungsschlitz (320) mit einer Bahn, die mindestens teilweise mit Nocken versehen ist, umfasst,
- der 2. Körper (314) mindestens einen Vorsprung (322) umfasst, der in dem Führungsschlitz (320) in Eingriff steht,
- die Bewegung der Bewegungsführung (318) aus der Halte in die Freigabeposition ein Gleiten des Führungsschlitzes (320) und eine Bewegung des mindestens einen Vorsprungs (322) entlang der mit Nocken versehenen Bahn bewirkt, wodurch mindestens ein Teil des 2. Körpers (314) von dem 1. Körper (312) getrennt wird.

6. Packung (100) nach einem der Ansprüche 2 bis 5, wobei der 1. Körper (312) und der 2. Körper (314) über ein Scharnier, vorzugsweise über ein Filmscharnier, verbunden sind.

7. Packung (100) nach einem der Ansprüche 1 bis 6, wobei der Vial-Halteraum (410) zum Halten einer Flüssigkeit ausgestaltet ist.

8. Packung (100) nach einem der Ansprüche 1 bis 7, wobei das Vial (400) eine Öffnung (412) hat, die mit einer Kappe oder einer entfernbaren Dichtung verschließbar ist.

9. Packung (100) nach einem der Ansprüche 1 bis 8, wobei der 1. Körper (312) mit einer Halteelement (324) versehen ist, die dazu ausgestaltet ist, die Bewegung des Implantats (230) in dem Lumen (222) des Zuführschafts (220) in der Richtung des distalen Endes (40) zu begrenzen.

10. Packung (100) nach Anspruch 9, wobei das Halteelement einen Ausstülpung umfasst, der dazu ausgestaltet ist, mindestens teilweise mit dem Lumen (222) des Zuführschafts (220) in Eingriff zu kommen.

11. Packung (100) nach einem der Ansprüche 2 bis 10, wobei der 1. Körper (312) und der 2. Körper (314) zusammenwirken, um mindestens einen Teil des Zuführschafts in einem Klemmbereich zu klemmen, so dass das Zuführschaftlumen (222) in dem Klemmbereich (312a, 314a) mindestens teilweise verengt ist, wodurch die Bewegung des Implantats (230) in einer proximalen (20) Richtung begrenzt wird.

12. Packung (100) nach einem der Ansprüche 1 bis 11, wobei der Einsatz (300) ferner einen oder mehrere Fluidverdrängungskörper (340) umfasst, die dazu ausgestaltet sind, während des Koppelns des Einsatzes (300) in den Vial-Halteraum (410) (wässrige) Lösung aus dem Vial-Halteraum (410) zu verdrängen.

13. Packung (100) nach Anspruch 12, wobei der Einsatz (300) ferner einen Überlaufauslass (342) umfasst, der für den Durchgang der aus dem Vial verdrängten Lösung ausgestaltet ist.

14. Packung (100) nach einem der Ansprüche 1 bis 13, ferner umfassend die Zuführschaftbaugruppe (200) nach Anspruch 1.

## Revendications

1. Emballage (100) pour maintenir et distribuer un ensemble formant une tige d'administration (200) pour l' administration d'un implant (230) pour le traitement d'un glaucome
- l'ensemble formant une tige d'administration (200) comprenant une tige d'administration (220) ayant une lumière (222) maintenant l'implant (230), et un adaptateur (210) conçu pour s'accoupler à un outil d'insertion (500),
- l'outil d'insertion (500) étant pourvu d'une tige d'évacuation (510) conçue pour être reçue par la lumière (222) de l'ensemble formant une tige d'administration, et d'un accouplement d'adaptateur (520) conçu pour s'accoupler avec l'adaptateur (210), la tige d'administration (220) et/ou la tige d'évacuation (510) pouvant être déplacées de sorte que l'implant (230) soit libéré de la lumière,
**caractérisé en ce que** l'emballage (100) comprend :
- un flacon (400) et une insertion (300) montée à l'intérieur d'un espace de maintien de flacon (410), l'insertion (300) comprenant :
- un corps de réception de force (308) conçu pour supporter l'adaptateur (210) pendant l'accouplement à l'outil d'insertion (500),
- un mécanisme de retenue de tige d'administration (310) conçu pour retenir de manière libérable la tige d'administration,
une application de force par l'outil d'insertion (500) pendant l'accouplement à l'adaptateur (210) actionnant le mécanisme de retenue de tige d'administration (310) pour libérer la tige d'administration (220) retenue pour un retrait de l'ensemble formant une tige d'administration (200) de l'insertion (300) et du flacon (400).

2. Emballage (100) selon la revendication 1, le mécanisme de retenue de tige d'administration (310) comprenant un 1er corps (312) et un 2nd corps (314) conçus pour coopérer afin de serrer au moins une partie de la tige d'administration (220).

3. Emballage (100) selon la revendication 2, le 1er corps (312) étant disposé en relation fixe par rapport au corps de réception de force (308), et le 2nd corps (314) étant conçu pour s'éloigner au moins partiellement lors de l'actionnement du mécanisme de retenue de tige d'administration (310) du 1er corps (312), libérant ainsi la tige d'administration (220) retenue.

4. Emballage (100) selon la revendication 3, le mécanisme de retenue de tige d'administration (310) comprenant en outre une goupille d'actionnement (316) et un guide de mouvement (318), une application de force par l'outil d'insertion pendant l'accouplement faisant avancer la goupille (316) et le guide de mouvement (318) d'une position de retenue à une position de libération, le guide de mouvement (318), accouplé de façon coulissante au 2nd corps (314), entraînant le mouvement du 2nd corps (314) d'une position de retenue à une position de libération.

5. Emballage (100) selon la revendication 4,
- le guide de mouvement (318) comprenant au moins une fente de guidage (320) ayant au moins une trajectoire partiellement camée,
- le 2nd corps (314) comprenant au moins une saillie (322) en prise dans la fente de guidage (320),
- un mouvement du guide de mouvement (318) de la position de retenue à la position de libération entraînant un coulissement de la fente de guidage (320) et un mouvement de l'au moins une saillie (322) le long de la trajectoire camée, séparant ainsi au moins une partie du 2nd corps (314) du 1er corps (312).

6. Emballage (100) selon l'une quelconque des revendications 2 à 5, le 1er corps (312) et le 2nd corps (314) étant raccordés par une charnière, de préférence par une charnière souple.

7. Emballage (100) selon l'une quelconque des revendications 1 à 6, l'espace de maintien de flacon (410) étant conçu pour retenir un liquide.

8. Emballage (100) selon l'une quelconque des revendications 1 à 7, le flacon (400) ayant une ouverture (412) pouvant être fermée par un capuchon ou un joint amovible.

9. Emballage (100) selon l'une quelconque des revendications 1 à 8, le 1er corps (312) étant pourvu d'un élément de retenue (324), conçu pour limiter un mouvement de l'implant (230) dans la lumière (222) de la tige d'administration (220) dans la direction de l'extrémité distale (40).

10. Emballage (100) selon la revendication 9, l'élément de retenue comprenant une protubérance conçue pour entrer au moins partiellement en prise avec la lumière (222) de la tige d'administration (220).

11. Emballage (100) selon l'une quelconque des revendications 2 à 10, le 1er corps (312) et le 2nd corps (314) coopérant afin de serrer au moins une partie de la tige d'administration dans une région de serrage de sorte que la lumière (222) de la tige d'administration dans la région de serrage (312a, 314a) soit au moins partiellement rétrécie, limitant ainsi le mouvement de l'implant (230) dans une direction proximale (20).

12. Emballage (100) selon l'une quelconque des revendications 1 à 11, l'insertion (300) comprenant en outre un ou plusieurs corps de déplacement de fluide (340) conçus pour déplacer une solution (aqueuse) à partir de l'espace de maintien de flacon (410) pendant l'accouplement de l'insertion (300) dans l'espace de maintien de flacon (410).

13. Emballage (100) selon la revendication 12, l'insertion (300) comprenant en outre une sortie de débordement (342) conçue pour le passage de la solution déplacée du flacon.

14. Emballage (100) selon l'une quelconque des revendications 1 à 13, comprenant en outre l'ensemble formant une tige d'administration (200) tel que défini dans la revendication 1.
